# EUROPEAN PATENT APPLICATION

(11) **EP 1 671 583 A1**
(43) Date of publication of application: **21.06.2006**
(21) Application number: 05027093.3
(22) Date of filing: 12.12.2005
(51) Int. Cl.: A61B 5/053

(54) **Body composition monitor**

(30) Priority: 15.12.2004 JP 2004362227
(71) Applicant: Omron Healthcare Co., Ltd., Kyoto 600-0084 (JP)
(72) Inventor: Tameo, Ashida, Omron Healthcare Co., Ltd, Kyoto-shi Kyoto 615-0084 (JP); Satoshi, Yamada, Omron Healthcare Co., Ltd, Kyoto-shi Kyoto 615-0084 (JP); Kiichiro, Miyata, Omron Healthcare Co., Ltd, Kyoto-shi Kyoto 615-0084 (JP)
(74) Representative: Kilian, Helmut

(57) **Abstract**

A body composition monitor includes a first input portion (6) for receiving input of personal data of each subject, a storage portion (26) for storing personal data in association with each subject, and a body composition monitor main unit (3) having a hold portion held with both hands of each subject. The body composition monitor main unit includes a second input portion (15-1, ... 15-4), arranged in such a position that the second input portion can be operated while the hold portion is held with both hands, for receiving an instruction to selectively read out one of the personal data.

## Description

### BACKGROUND OF THE INVENTION

### Field of the Invention

The present invention relates to a body composition monitor for measuring a bioelectrical impedance and determining body composition such as body fat mass and rate, fat free mass and rate, visceral fat area and mass, muscle mass and rate, total body water, and the like based on the measured bioelectrical impedance.

### Description of the Background Art

Generally, a body composition monitor including hold portions provided with a current application electrode and a voltage sensing electrode on the left and right of a body composition monitor main unit having a display portion and an operation portion has been known, in which bioelectrical impedance is measured by holding the hold portions with both hands, and body fat mass and rate and the like are determined based on the measured bioelectrical impedance.

It has conventionally been disclosed with regard to a body composition monitor of this type that an end portion of a current application electrode on a right-hand thumb side is replaced with a measurement start switch in electrode holding portions provided at opposing ends of an operation main unit, so that the measurement start switch can be operated while both hands hold the current application electrodes and voltage sensing electrodes (for example, see Japanese Patent Laying-Open No. 10-174680). In addition, a body composition monitor including grips each provided with a current application electrode and a voltage sensing electrode on opposing sides of a body composition monitor main unit and also including a measurement start switch in a connection portion between an upper end of the right grip and the body composition monitor main unit, measurement being performed by gripping the electrodes in the grips with both hands, has been disclosed (for example, see Japanese Patent Laying-Open No. 10-179534).

Recently, a body composition monitor has been developed, in which an operation portion includes a plurality of personal data read-out switches, personal data such as male/female, age, height, and the like is stored by a user in a manner corresponding to a switch number, and in measurement, the user operates the personal data read-out switch corresponding to which his/her personal data has been registered, so as to call the personal data and start measurement.

The reading-out switch of this type is arranged in an area in an operation main portion where other switches are provided, and the reading-out switch is located below the display portion. Therefore, the reading-out switch is not easily operated while the hold portion is held. Specifically, while the body composition monitor main unit is supported by one hand without the hold portion being held, an input operation is performed with the other hand, and thereafter the hold portion is held. In other words, the personal data reading-out switch should be operated before the hold portion is held, which has been burdensome. Operability of the reading-out switch has thus been poor. According to Japanese Patent Laying-Open Nos. 10-174680 and 10-179534, one measurement start switch is provided in an area different from an area of the operation portion. These publications, however, are silent about a plurality of personal data reading-out switches for reading out specific personal data and arrangement of these switches in an area different from the area of the operation portion.

### SUMMARY OF THE INVENTION

The present invention has been made, paying attention to the above-described problems. An object of the present invention is to provide a body composition monitor attaining excellent operability with regard to a plurality of switches for specifying an individual.

A body composition monitor according to the present invention measures bioelectrical impedance of a subject. The body composition monitor includes a first input portion, a storage portion, a body composition monitor main unit, and a read-out portion. The first input portion serves to receive input of personal data such as male/female, age, height, and the like of the subject. The storage portion stores the personal data in association with each subject. The body composition monitor main unit has a hold portion held with both hands of each subject. The body composition monitor main unit includes a plurality of first electrodes, a plurality of second electrodes, and a second input portion. The plurality of first electrodes are arranged on the hold portion, and come in contact with both hands of the subject for application of a current. The plurality of second electrodes are arranged on the hold portion, and sense a voltage while the current is applied. The second input portion is arranged in such a position that it can be operated while the hold portion is held with both hands, and receives an instruction to selectively read out one of the personal data from the storage portion. The read-out portion reads out one of personal data, in response to the instruction.

Preferably, the body composition monitor main unit includes a main portion having a display portion for displaying body composition of the subject, the hold portion has grips extending toward left and right from the main portion respectively, and the second input portion is provided between the grips and in the vicinity of one electrode of the first and second electrodes provided in the grips.

Preferably, the second input portion includes a first switch and a second switch, and the first switch is provided in the vicinity of leftmost electrode of the first and second electrodes that are arranged on the grip on the right, and the second switch is provided in the vicinity of rightmost electrode of the first and second electrodes that are arranged on the grip on the left.

Preferably, the second input portion is provided in an area where the first and second electrodes are arranged.

Preferably, the body composition monitor main unit has a first surface, the first input portion has a first switch, the second input portion includes a plurality of second switches, and the first and second switches are provided on the first surface.

Alternatively, the body composition monitor main unit may have a first surface and a second surface, the first input portion has a first switch, the second input portion includes a plurality of second switches, and the first switch may be provided on the first surface and the second switches may be provided on the second surface.

Preferably, the second input portion may include a first switch and a second switch, and the first switch may be provided on a left side surface of the body composition monitor main unit, and the second switch may be provided on a right side surface of the body composition monitor main unit.

Preferably, the first input portion and the storage portion are included in the body composition monitor main unit.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is an external view of a body composition monitor according to one embodiment of the present invention.
Fig. 2 is a rear view of a scale included in the body composition monitor according to the embodiment.
Fig. 3 is an enlarged external view of a body composition monitor main unit of the body composition monitor according to the embodiment.
Fig. 4 is a block diagram showing a circuit configuration of the body composition monitor according to the embodiment.
Figs. 5A and 5B illustrate an operation of a switch for specifying an individual in the body composition monitor according to the embodiment.
Figs. 6A and 6B illustrate an operation of a guest switch in the body composition monitor according to the embodiment.
Fig. 7 is a flowchart illustrating a processing operation when body composition is measured with the body composition monitor according to the embodiment.
Fig. 8 shows a partial rear surface of a body composition monitor main unit of a body composition monitor according to another embodiment of the present invention.
Fig. 9 shows a body composition monitor main unit of a body composition monitor according to yet another embodiment of the present invention.

### DESCRIPTION OF THE PREFERRED EMBODIMENTS

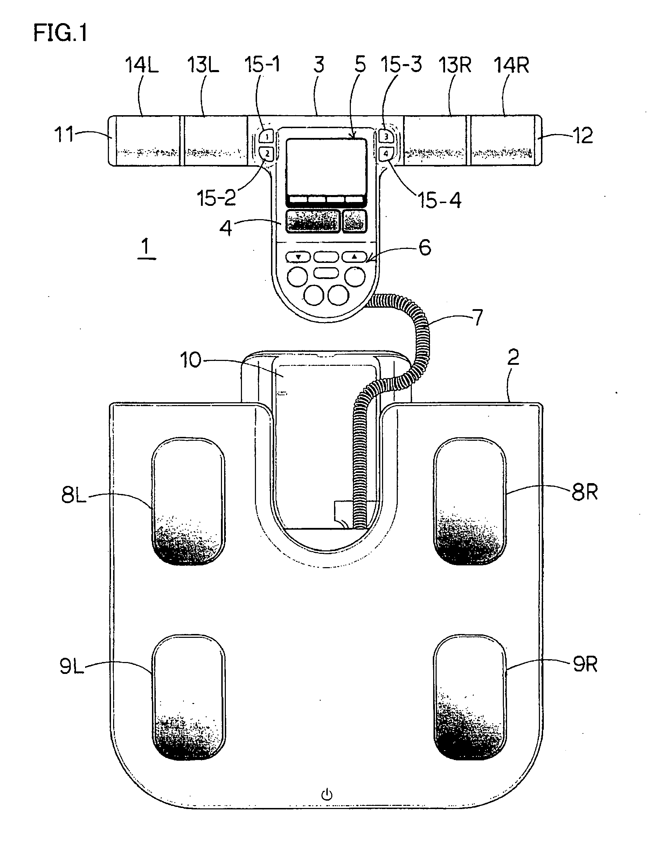

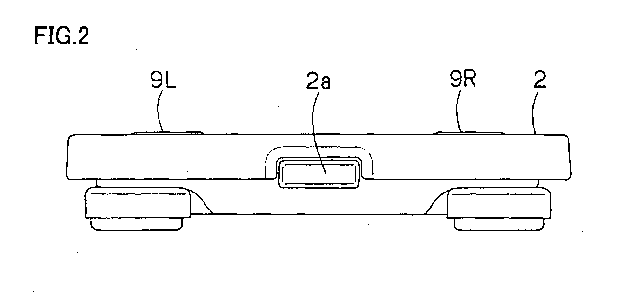

The present invention will be described hereinafter with reference to an embodiment. Fig. 1 is an external view of a body composition monitor according to one embodiment of the present invention, and Fig. 2 is a rear view of a scale of the body composition monitor. A body composition monitor 1 according to the present embodiment is implemented by a scale 2 and a body composition monitor main unit 3. Scale 2 and body composition monitor main unit 3 are electrically connected to each other by a freely extendable cord 7.

Scale 2 contains a strain sensor for measuring body weight and includes on its surface left-foot electrodes 8L, 9L and right-foot electrodes 8R, 9R for measuring bioelectrical impedance. Left-foot electrode 8L and right-foot electrode 8R serve as electrodes for applying a high-frequency current. Left-foot electrode 9L and right-foot electrode 9R serve as electrodes for sensing a voltage. In addition, scale 2 includes a power switch 2a on its rear surface. Scale 2 includes an accommodation portion 10 for accommodating body composition monitor main unit 3, in front of left-foot electrode 8L and right-foot electrode 8R and between the same.

Body composition monitor main unit 3 includes a main portion 4, a left grip 11 and a right grip 12, and main portion 4 includes a display portion 5 and an operation portion 6. On opposing sides of main portion 4, left grip 11 and right grip 12 are integrally formed like a handle of a bicycle. Left-hand electrodes 13L, 14L and right-hand electrodes 13R, 14R are provided in left grip 11 and right grip 12, respectively. Left-hand electrode 13L and right-hand electrode 13R serve as electrodes for applying a high-frequency current, while left-hand electrode 14L and right-hand electrode 14R serve as voltage sensing electrodes that sense a voltage generated between body parts as a result of application of the high-frequency current. These electrodes 13L, 14L, 13R, and 14R are internally connected to a circuit portion in main portion 4.

According to the present embodiment, though handle-like left grip 11 and right grip 12 are provided in body composition monitor main unit 3, any member that can be held with both hands of the subject may be provided, instead of a handle-like member.

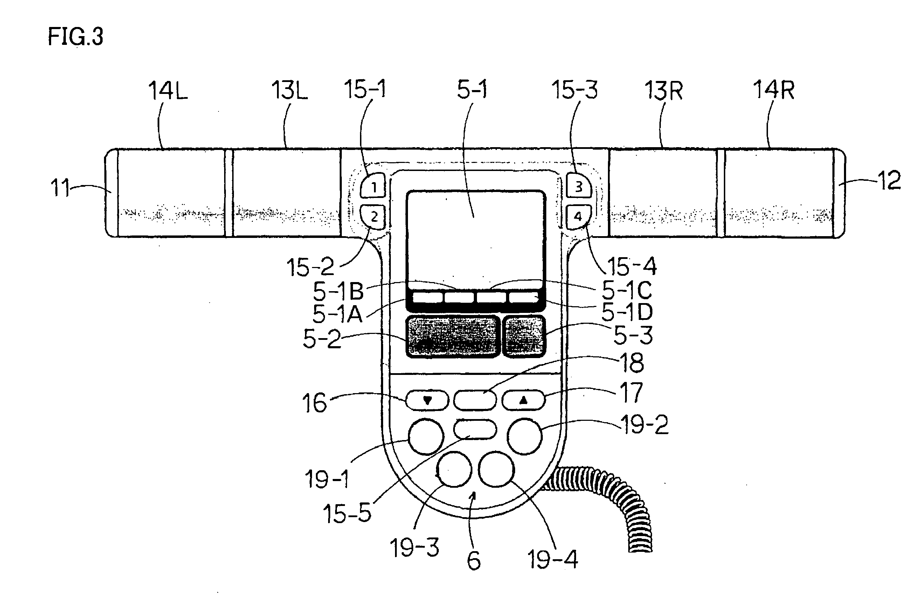

Fig. 3 is an enlarged view of body composition monitor main unit 3. Display portion 5 includes a display portion 5-1 for displaying main data such as a setting value and a measurement value and levels such as low, normal, slightly high, and high, a display portion 5-2 for displaying BMI and basal metabolism (kcal), and a display portion 5-3 for displaying body age (year(s) of age). In the present embodiment, areas 5-1A, 5-1B, 5-1C, and 5-1D for indicating levels of measured values such as low, normal, slightly high, and high are provided from left to right below display portion 5-1. Operation portion 6 includes a guest switch 15-5, a DOWN switch 16, an UP switch 17, a setting switch 18, a body weight switch 19-1, a skeletal muscle level switch 19-2, a body fat rate switch 19-3, and a visceral fat level switch 19-4. In addition, in an area different from a switch arrangement area in operation portion 6, switches 15-1, 15-2 for reading out personal data of subjects are provided between left-hand electrode 13L and display portion 5-1, in the vicinity of left-hand electrode 13L for current application, and switches 15-3, 15-4 for reading out the personal data of subjects are provided between right-hand electrode 13R and display portion 5-1, in the vicinity of right-hand electrode 13R for current application.

Switches 15-1, 15-2, 15-3, and 15-4 serve to receive instructions to read out personal data of an individual 1, an individual 2, an individual 3, and an individual 4 whose personal data have been registered in advance. The body composition monitor according to the present embodiment is most characterized in that these switches 15-1, ... 15-4 for reading out the personal data are arranged not in the switch arrangement area in operation portion 6 but between left-hand electrode 13L and right-hand electrode 13R, in the vicinity of left-hand electrode 13L for current application or in the vicinity of right-hand electrode 13R for current application.

Guest switch 15-5 is used when a person whose personal data has not been registered, i.e., a guest, uses the present device. DOWN switch 16 and UP switch 17 are operated when a marker or a value is lowered and raised respectively. Setting switch 18 is used when a variety of items are set. Body weight switch 19-1 is operated when a "body weight" is displayed out of measurement results. Skeletal muscle level switch 19-2 is operated when a "skeletal muscle level" is displayed out of measurement results. Body fat rate switch 19-3 is operated when a "body fat rate" is displayed out of measurement results. Visceral fat level switch 19-4 is operated when a "visceral fat level" is displayed out of measurement results. In other words, switches 19-1, 19-2, 19-3, and 19-4 serve as confirmation switches for confirming the measurement result.

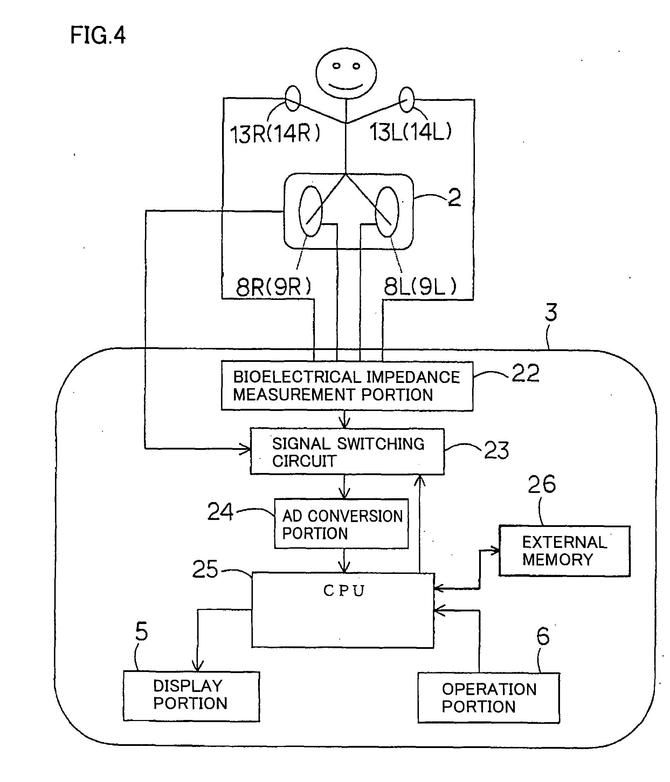

Fig. 4 shows a circuit configuration of body composition monitor 1 according to the present embodiment. As described above, scale 2 of body composition monitor 1 contains the strain sensor for measuring body weight. In addition, right-foot electrode 8R and left-foot electrode 8L for measuring bioelectrical impedance serve as electrodes for applying a high-frequency current, while right-foot electrode 9R and left-foot electrode 9L serve as electrodes for sensing a voltage. Body composition monitor main unit 3 includes display portion 5, operation portion 6, a bioelectrical impedance measurement portion 22, a signal switching circuit 23, an A/D conversion portion 24, a CPU 25, and an external memory 26.

Bioelectrical impedance measurement portion 22 connected to right-foot electrode 8R (9R), left-foot electrode 8L (9L), right-hand electrode 13R (14R), and left-hand electrode 13L (14L) detects a voltage between voltage sensing electrodes, so as to measure bioelectrical impedance. Bioelectrical impedance measurement portion 22 can measure impedance of the whole body and of each region such as trunk, leg, arm, and the like. Signal switching circuit 23 outputs a signal of body weight measured by scale 2 and the bioelectrical impedance measured by bioelectrical impedance measurement portion 22, in a manner switched in response to a switch command from CPU 25. A/D conversion portion 24 converts a signal output from signal switching circuit 23 from analog to digital, and inputs the resultant signal to CPU 25. CPU 25 attains a function to calculate body fat mass and rate, fat free mass and rate, visceral fat area and mass, muscle rate, and other body composition with regard to the whole body and each region, based on the personal data such as male/female, age and height input through operation portion 6 in advance, the body weight input from scale 2, and the bioelectrical impedance input from bioelectrical impedance measurement portion 22. External memory 26 includes a personal data storage area, in which "age", "male/female", "height" and the like corresponding to each individual 1, individual 2, individual 3, and individual 4 can be registered. In addition, external memory 26 can store body composition data for each individual that was measured in the past. The personal data storage area may be provided in a memory within CPU 25.

In body composition monitor 1 according to the present embodiment, personal data of a plurality of users (subjects) can be registered in advance. Registration is performed in the following manner. Power switch 2a is turned on, and a personal data read-out switch, corresponding to which registration is to be made, is pressed while "0.0kg" is displayed on display portion 5-1. For example, personal data read-out switch 15-1 is pressed, so that selected individual number 1 is displayed on display portion 5-1. Thereafter, setting switch 18 is pressed to determine the individual number. Thereafter, "age" is input and setting switch 18 is pressed, in order to register the "age". Then, "male/female" is input and setting switch 18 is pressed, in order to register the "male/female". Finally, "height" is input and setting switch 18 is pressed, in order to register the "height". As described above, "age", "male/female" and "height" are registered in external memory 26 for each individual 1, individual 2, individual 3, and individual 4. Personal data such as "waist size (length around the waist)" may further be registered.

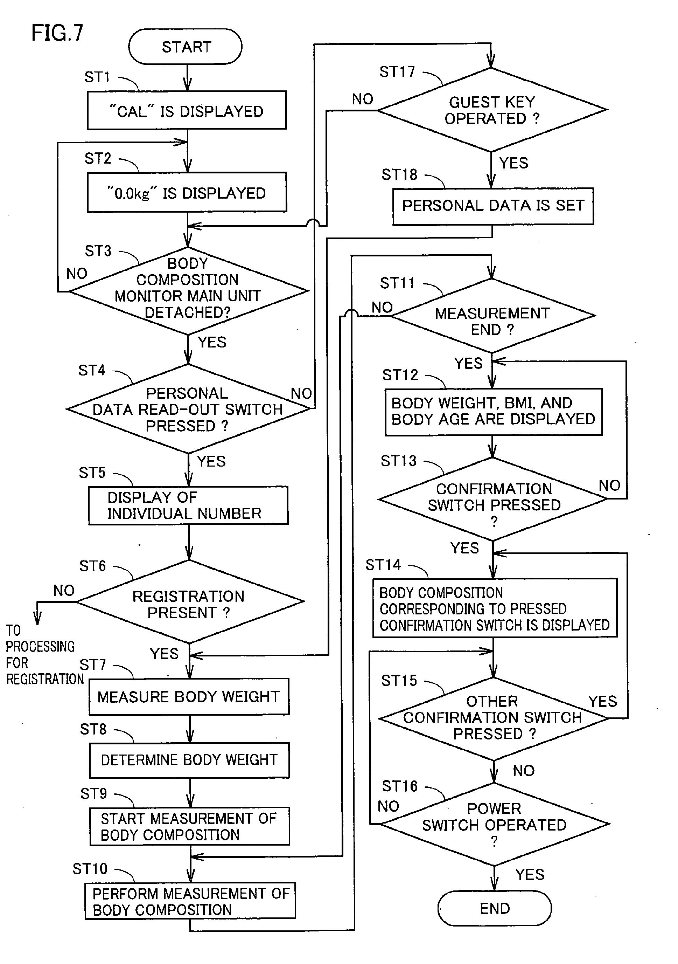

The processing operation when body weight and body composition are measured with body composition monitor 1 according to the present embodiment will now be described with reference to the flowchart shown in Fig. 7. When the user turns on power switch 2a for starting measurement, the processing operation is started. At step ST1, "CAL" is displayed in a blinking manner on display portion 5-1. During blinking, the device checks whether measurement can correctly be conducted (calibration).

When calibration is finished, the process proceeds to step ST2, at which "0.0k" is displayed on display portion 5-1. If the user steps on or moves the scale before "0.0kg" is displayed, "Err (error)" is displayed. Then, the process proceeds to step ST3.

At step ST3, whether body composition monitor main unit 3 has been detached from accommodation portion 10 is determined. Display of "0.0kg" as a result of the processing at steps ST2 and ST3 is continued until body composition monitor main unit 3 is detached. The user is supposed to detach body composition monitor main unit 3 from accommodation portion 10 when he/she sees the display of "0.0kg". When it is determined that body composition monitor main unit 3 has been detached, the process proceeds to step ST4.

At step ST4, whether the personal data read-out switch has been pressed is determined. If the personal data read-out switch is not pressed for a prescribed period, the process proceeds to step ST17. On the other hand, if any of switches 15-1, ... 15-4 is pressed, the process proceeds to step ST5.

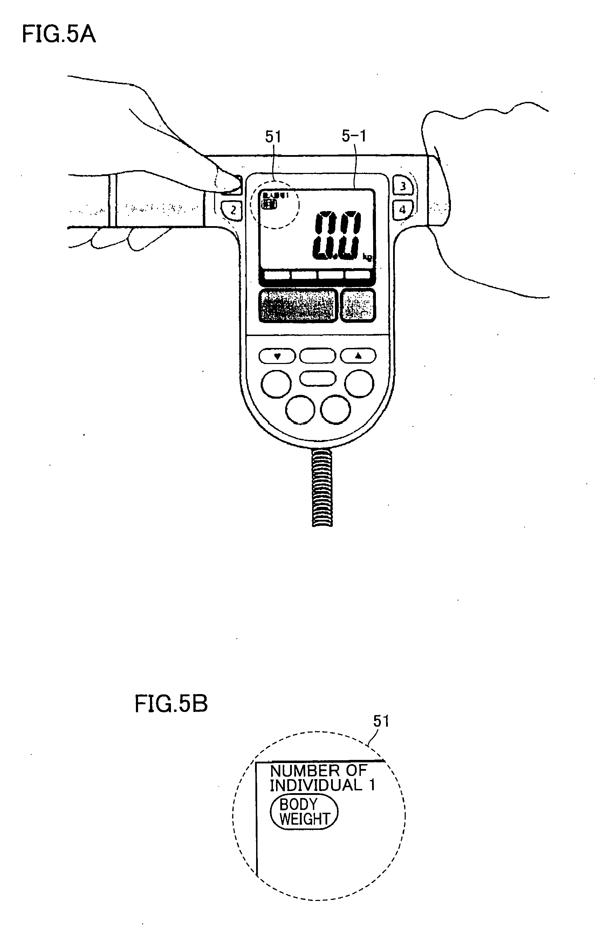

At step ST5, the individual number corresponding to the pressed switch is displayed on display portion 5-1 in a blinking manner. In order to call personal data, the user presses a personal data read-out switch corresponding to which his/her own personal data has been registered, out of personal data read-out switches 15-1, ... 15-4. As a result, the individual number corresponding to his/her own personal data is displayed on display portion 5-1. For example, if selection of individual number "1" is to be made, switch 15-1 is pressed with a thumb while electrodes 13L, 14L in the left grip are gripped with the left hand as shown in Fig. 5A. Fig. 5B is an enlarged view of a portion 51 encircled with a dashed line in Fig. 5A. Referring to Fig. 5B, individual number "1" and characters "body weight" are shown on display portion 5-1. If personal data corresponding to the selected individual number is not registered, the individual number is not shown and a dash (bar) is displayed.

Successively, the process proceeds to step ST6. At step ST6, whether the personal data corresponding to the pressed switch has been registered is determined. If the personal data has not been registered, the process proceeds to processing for registration. On the other hand, if the personal data has been registered, the process proceeds to step ST7. As the user is on scale 2 at this stage, measurement of body weight is started at step ST7. When measurement of body weight ends, the process proceeds to step ST8, at which the measured body weight is shown on display portion 5-1 in a manner blinking twice, for confirmation of the body weight. Then, the process proceeds to step ST9.

At step ST9, measurement of body composition is started. In addition, at step ST10, measurement of body composition is performed. During measurement, progress of measurement is shown with a bar indicator on display portion 5-1. When it is determined at step ST11 that measurement of body composition has ended, the process proceeds to step ST12. At step ST12, measured body weight is displayed on display portion 5-1, and BMI and body age are displayed on display portions 5-2 and 5-3 respectively. Then, the process proceeds to step ST13. At step ST13, whether the confirmation switch, that is, any one of switches 19-1, ... 19-4 has been pressed is determined. If any one of switches 19-1, ... 19-4 is pressed, the process proceeds to step ST14. At step ST14, the body composition corresponding to the pressed confirmation switch is displayed. For example, if body fat rate switch 19-3 is pressed, a value of the body fat rate and evaluation of the body fat rate are displayed on display portion 5-1. In evaluating the body fat rate, a bar indicator is used to show the level such as low, normal, slightly high, and high. Then, the process proceeds to step ST15. At step ST15, whether other confirmation switch has been pressed is determined. If any other confirmation switch is pressed, the process returns to step ST14, and the body composition corresponding to the pressed confirmation switch is displayed. If the confirmation switch is not pressed for a prescribed period at step ST15, the process proceeds to step ST16. When power switch 2a is pressed at step ST16, the process ends.

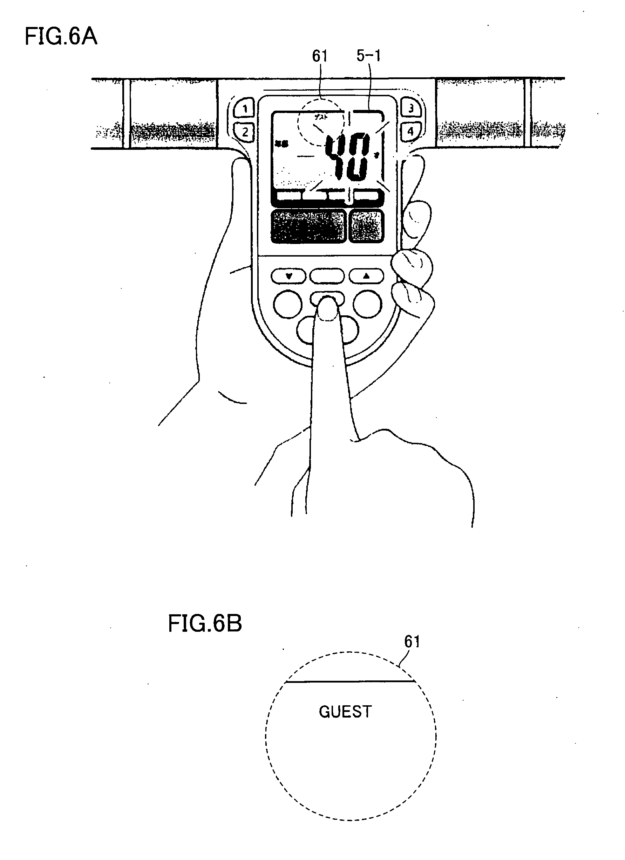

If guest switch 15-5 is pressed at step ST4 as shown in Fig. 6A instead of personal data read-out switch 15-1, ... 15-4, determination as YES is made at step ST17 and the process proceeds to step ST18. Fig. 6B is an enlarged view of a portion 61 encircled with a dashed line in Fig. 6A. Referring to Fig. 6B, a character "guest" is shown on display portion 5-1. At step ST18, personal data such as age, male/female and height is set, and the process proceeds to step ST7, at which body weight is measured.

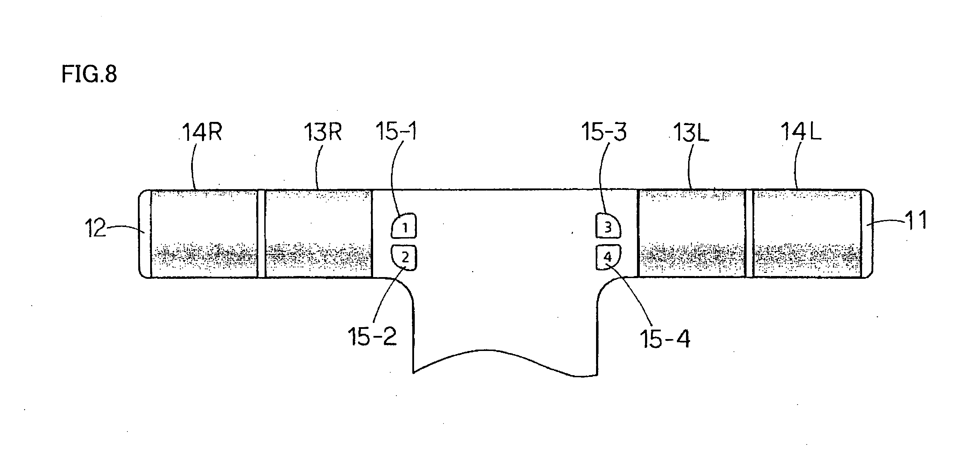

In the embodiment shown in Figs. 1 and 3, an example in which switches 15-1, ... 15-4 for specifying an individual are arranged on the surface of body composition monitor main unit 3 has been shown. According to another embodiment as shown in Fig. 8, however, switches 15-1, 15-2 may be arranged vertically in the vicinity of right-hand electrode 13R on the rear surface of body composition monitor main unit 3 and switches 15-3, 15-4 may be arranged vertically in the vicinity of left-hand electrode 13L on the same. Alternatively, respective pairs of switches 15-1 and 15-2 and switches 15-3 and 15-4 may be arranged horizontally. Further alternatively, respective pairs of switches 15-1 and 15-2 and switches 15-3 and 15-4 may be arranged diagonally. A pair of switches 15-1, 15-2 and a pair of switches 15-3, 15-4 arranged on the rear surface in the above-described manner are operated by right and left forefinger respectively, while the left and right grips are gripped with left and right hands respectively.

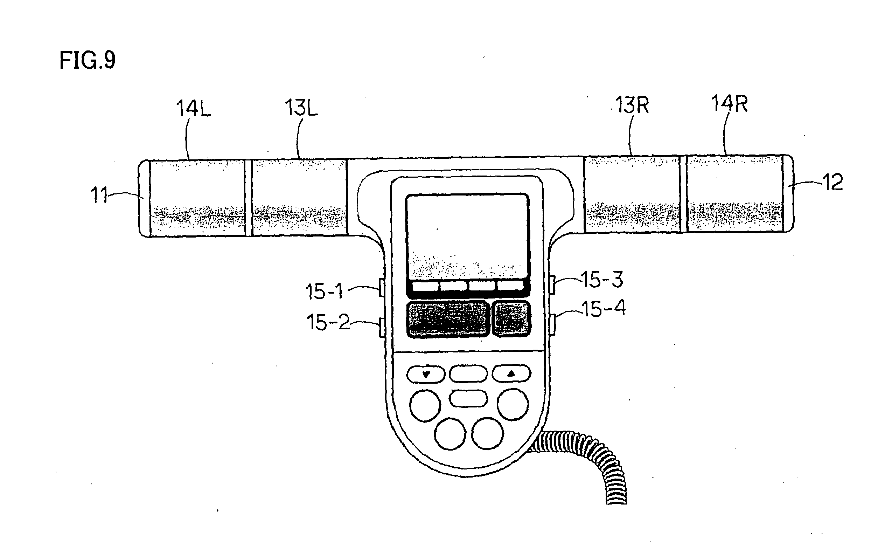

In addition, according to yet another embodiment as shown in Fig. 9, personal data read-out switches 15-1, 15-2 and personal data read-out switches 15-3, 15-4 may be provided on a right side surface and a left side surface of main portion 4 of body composition monitor main unit 3, respectively. In this case, the switch is pressed with the tip end of the left or right thumb.

Moreover, according to yet another embodiment, personal data read-out switches 15-1, ... 15-4 may be arranged within an area where left-hand electrodes 13L, 14L and right-hand electrodes 13R, 14R are arranged, with a distance from each electrode. In this case, each electrode in which a switch is arranged is formed to include an opening where the switch is to be arranged.

According to the present invention, switches 15-1, ... 15-4 for receiving an instruction to read out personal data are provided between left and right grips 11, 12 and in the vicinity of left and right electrodes 13L, 13R, 14L, and 14R, such that these switches can be operated while grips 11, 12 are held with both hands. At the time of measurement, grips 11, 12 are held and body composition monitor main unit 3 accommodated in scale 2 is taken out. Accordingly, measurement can be conducted without moving one's hands off. Therefore, during measurement, a contact state between hands and electrodes 13L, 13R, 14L, and 14R is made stable and measurement error is not caused. In addition, as switches 15-1, ... 15-4 can be operated while grips 11, 12 are held, operability is improved.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A body composition monitor for measuring bioelectrical impedance of subjects, comprising:
a first input portion (6) for receiving input of personal data of each said subject;
a storage portion (26) for storing said personal data in association with each said subject;
a body composition monitor main unit (3) having a hold portion held with both hands of each said subject, said body composition monitor main unit including
a plurality of first electrodes (13L, 13R) arranged on said hold portion for being in contact with said both hands respectively and applying a current,
a plurality of second electrodes (14L, 14R) arranged on said hold portion for sensing a voltage while said current is applied, and
a second input portion (15-1,···,15-4), arranged in such a position that said second input portion can be operated while said hold portion is held with said both hands, for receiving an instruction to selectively read out one of said personal data from said storage portion; and
a read-out portion (25) for reading out said one of personal data in response to said instruction.

2. The body composition monitor according to claim 1, wherein
said body composition monitor main unit includes a main portion (4) having a display portion for displaying body composition of said subjects,
said hold portion has grips (11, 12) extending toward left and right from said main portion respectively, and
said second input portion is provided between said grips and in vicinity of one electrode of said first and second electrodes provided in said grips.

3. The body composition monitor according to claim 2, wherein
said second input portion includes a first switch and a second switch, and
said first switch is provided in vicinity of leftmost electrode of said first and second electrodes that are arranged on said grip on the right, and said second switch is provided in vicinity of rightmost electrode of said first and second electrodes that are arranged on said grip on the left.

4. The body composition monitor according to claim 1, wherein
said second input portion is provided in an area where said first and second electrodes are arranged.

5. The body composition monitor according to claim 1, wherein
said body composition monitor main unit has a first surface,
said first input portion has a first switch (16 to 18, 19-1 to 19-5),
said second input portion includes a plurality of second switches, and
said first and second switches are provided on said first surface.

6. The body composition monitor according to claim 1, wherein
said body composition monitor main unit has a first surface and a second surface,
said first input portion has a first switch (16 to 18, 19-1 to 19-5),
said second input portion includes a plurality of second switches, and
said first switch is provided on said first surface and said second switches are provided on said second surface.

7. The body composition monitor according to claim 1, wherein
said second input portion includes a first switch and a second switch, and
said first switch is provided on a left side surface of said body composition monitor main unit, and said second switch is provided on a right side surface of said body composition monitor main unit.

8. The body composition monitor according to claim 1, wherein
said first input portion and said storage portion are included in said body composition monitor main unit.
